## Europäisches Patentamt
## European Patent Office
## Office européen des brevets

(19)

(11) Publication number: **0 026 574**
**B1**

(12)

# EUROPEAN PATENT SPECIFICATION

(45) Date of publication of patent specification: **04.01.84**

(21) Application number: **80302891.9**

(22) Date of filing: **21.08.80**

(51) Int. Cl.³: **C 08 J 3/12, B 29 B 1/12,
A 61 K 9/14, B 01 J 47/00**

(54) **The production of powdered resin and the powdered resin so produced.**

(30) Priority: **29.08.79 GB 7929908**

(43) Date of publication of application:
**08.04.81 Bulletin 81/14**

(45) Publication of the grant of the patent:
**04.01.84 Bulletin 84/1**

(84) Designated Contracting States:
**AT BE CH DE FR GB IT LI NL SE**

(56) References cited:
**GB - A - 733 987
GB - A - 947 957
GB - A - 950 218
GB - A - 1 079 131
GB - A - 1 286 949**

**Chemical Engineer's Handbook J.H. Perry
McGraw Hill New York 1941**

**The file contains technical information
submitted after the application was filed and not
included in this specification**

(73) Proprietor: **Rohm and Haas Company
Independence Mall West
Philadelphia, Pennsylvania 19105 (US)**

(72) Inventor: **Pirotta, Marico Giuseppe
Piazza Vesuvio 23
Milan (IT)**
Inventor: **Garbagnati, Giberto
Via San Vittore 40
Milan (IT)**

(74) Representative: **Angell, David Whilton et al,
Rohm and Haas Company Patent Department
Chesterfield House Bloomsbury Way
London WC1A 2TP (GB)**

Courier Press, Leamington Spa, England.

# 0 026 574

## The production of powdered resin and the powdered resin so produced

This invention is concerned with the production of powdered resin, particularly from synthetic polymeric adsorbent or ion exchange resin beads, and the powdered resin so produced.

It is known, for example from Schultz and Crook, Ind. and Eng. Chem. Product Research and Development, Vol. 7, No. 2, June 1968 pages 120 to 125 to be advantageous for some uses that a synthetic polymeric adsorbent or ion exchange resin have a particle size of up to 44 $\mu$m average particle diameter. In that disclosure there are demonstrated so-called ultrafine resins, 0.5 to 1.5 $\mu$m particle diameter, and so-called micropowder resins, particle diameter 25 to 44 $\mu$m are also mentioned. It is also disclosed that when the resins are for oral consumption the higher particle sizes are less attractive as they impart unpleasant grittiness in the mouth and tend to form non-stable, settling aqueous dispersions.

Ion exchange and adsorbent resin beads produced by conventional suspension polymerization processes generally have a particle size around 600 $\mu$m. Many attempts have been made to reduce this particle size by pulverising such beads. For example resin beads have been dried, removing entrapped water deriving from the aqueous suspension polymerization medium, and impacted by air pressure against a grid. Alternatively undried resin has been milled, dried and milled again. Such processes, however, all have disadvantages. For example in some cases it has been found that no matter how many passes the resin makes through the grinding mill the particle size cannot be reduced to a sufficiently low level. For effective size reduction in some mills the resin must be dried to avoid the lubricating effect of moisture. This is why, in the prior art process described above, the second grinding cycle is carried out on dried resin. However, this often has the disadvantage that the temperature in the mill rises, sometimes to such an extent that the resin may begin to degrade.

GB—950218 discloses the pulverizing of natural or synthetic resins, especially ethylene polymers, in the presence of water, as a lubricant and heat remover, using any grinding or pulverising equipment. However this patent is not addressed to the comminution of ion exchange or adsorbent resins. Further it does not teach the importance of the introduction of strain within the particles of such resin during comminution by the presence of free water or organic solvent or the importance of using a rotary attrition mill.

We have now found a process wherein beads of synthetic polymeric ion exchange or adsorbent resin may be efficiently and effectively comminuted without excessive temperature increase to such a low particle size that they form stable dispersions and to not have a gritty taste in the mouth.

This invention comprises a process for the comminution of particles of synthetic polymeric ion exchange or adsorbent resin wherein beads, or fragmented beads thereof are swollen or shrunk by contact with water or an organic solvent in a slurry therewith to introduce strain withtn the resin particle and are subsequently ground as a slurry in a rotary attrition mill. There is thus provided, according to one aspect of the invention, a process for the comminution of beads of synthetic polymeric ion exchange or adsorbent resin which comprises swelling or shrinking the beads, which have optionally been subjected to prefragmentation without drying, by contacting them for at least 0.5 minutes, and in the case of cholestyramine preferably at least 5 minutes, with water or an organic solvent adjusting, if necessary, the ratio of liquid (that is to say solvent or water which is not entrapped by the resin) to resin to at least 0.3:1, preferably at least 1:1 by weight and grinding the resulting slurry in a rotary attrition mill.

The rotary attrition mills suitable for use in the process of the invention are those mills which can be regarded as modern counterparts of the early bulkstone mill. Stones in the early mills are replaced by discs or cones or cylinders of metal or abrasive grinding material which rotate relative to each other. Grinding takes place between the cones, cylinders or plates in a horizontal or vertical plane. The grinding surfaces are generally shaped to come closer together along the path through which the material being ground passes. The space between the grinding surfaces is, however, adjustable and, without adjustment, does not vary during grinding. The grinding surfaces may carry projections such as teeth or spikes to aid in the grinding operation particularly at the coarser levels of grinding.

Thus in the preferred rotary attrition mills useful in the invention the resin particles are sheared and cut by passage through a predetermined grinding gap between relatively rotating surfaces.

As mentioned, the resin beads may be prefragmented before being subjected to the swelling/shrinking treatment. Additionally, after the swelling/shrinking step, the first stage of the comminution of the beads or prefragmented beads, (collectively called particles) may be carried out in a mill other than a rotary attrition mill, for example a ball mill.

The processes of this invention may be used to produce a ground resin which, when in the wet swollen state, has 90% by weight and/or number below 30 $\mu$m, preferably all below 30 $\mu$m, most preferably 90% by weight and/or number below 10 $\mu$m, in average particle diameter. When dry such a resin will have 90% by weight and/or number below 15 $\mu$m most preferably 90% by weight and/or number below 5 $\mu$m in average particle diameter. Measurement of particle size is by Coulter Counter technique using an isotonic solution.

2

The process is particularly suitable for comminuting nitrogen-containing polymers which have, *inter alia*, bile-acid fixing and triglyceride fixing ability. Such polymers are disclosed in U.K. Patent Specifications 929,391, 1,286,949 and 1,446,352. Such materials, commercially are generally strongly basic styrenedivinylbenzene aminated ion exchange resins in the chloride form. They may be gel or macroreticular resins but are generally gel. These so-called cholestyramine resins usually have a bead or chip size above 325 U.S. Standard Sieve series. Useful cholestyramine bead or chip resins are described in British Patent 929,391. The minimum properties of such resins, e.g. glycocholate capacity, are specified in the U.S. Pharmacopeia.

As indicated above, we believe that when resin particles, before comminution, are treated as indicated with water or organic solvent shrinking or swelling of the resin results to cause strain within the resin particle which significantly aids in the grinding step. Thus, although the resin beads may be fragmented before this swelling or shrinking step they should not be so small that such internal strain will not be caused. A minimum particle size for the, optionally prefragmented, resin beads used as starting material in the process of the invention is preferably 10 $\mu$m, most preferably 50 $\mu$m.

The contact time with the water or organic solvent should preferably be at least sufficient to cause the beads to shrink to 95% or swell to 105% of their dry size, preferably swell to 10 times dry size. This will not usually be achieved in less than 0.5 minutes. A preferred contact time is at least 15 minutes, most preferably 30 to 90, usually about 60 minutes.

In a preferred process of the invention the resin particles are treated, during the shrinking/swelling step, with a material in the water or organic solvent medium, which will induce osmotic shock and so increase the strain in the resin particle. An example of a suitable solute is sugar when the contact medium is aqueous. Other candidate solutes are sodium chloride or inorganic acids such as hydrochloric or bases such as sodium hydroxide or organic salts, alcohols, glycols, carbonyl compounds for aqueous media and mineral oils, hydrocarbons, chlorinated hydrocarbons, alcohols, glycols, carbonyl compounds for organic media although any solute which will not react with the resin and, preferably, has a high molecular weight would be suitable. These solutes are preferably present in the contact medium at concentrations of 5 to 50% by weight.

When the ground resin is to be used for pharmaceutical applications it is important to choose grinding surfaces which will not shed undesirable materials into the ground resin. We have found that corundum surfaces are acceptable in this respect. In one suitable mill employing corundum stones, the product to be treated is passed through a grinding gap between two horizontally or vertically mounted grinding stones, one of which rotates and the other of which is stationary. The resin particles are thus subjected to crushing on their way from the inlet at the centre of the stones to the outlet at the periphery, at numerous shearing points. The grinding gap between the stones is adjustable, preferably even during operation and preferably by such precise amounts as 5 microns. Suitable mills are preferably provided with grinding element cooling capability.

The grinding step is preferably carried out with a grinding gap of 0.001 to 0.01 mm, preferably about 0.005 mm, at a pressure of 2 to 40 kg/cm² (1 kg/cm²=0.98 bar), preferably about 5 kg/cm² (1 kg/cm²=0.98 bar) and a relative speed between the grinding surfaces of 2,000 to 25,000 R.P.M., preferably about 5,800 R.P.M.

As stated above this information is particularly suitable for the production of ground cholestyramine resin. Carbonaceous adsorbents can also be efficiently ground by this process. Among ion exchange resins and polymeric adsorbents, particularly suitable are those based on styrenedivinylbenzene resin beads which, when ground, may be useful in the fields of diagnostic, dietetic, pharmaceutical, alimentary, agricultural, veterinary and water treatment.

This invention will now be described, for the purposes of illustration only, in the following Example.

Example 1

To 10 kg cholestyramine wet beads; average particle size 600 $\mu$m moisture 75%, exchange capacity=4.2 meq/g dry (Cl⁻) spec. gravity 0.688; were added 40 kg deionized sterile water. The mixture was agitated for 5 minutes, left to stand for 60 minutes, stirred again and fed to a mill using a high pressure slurry pump. The mill used was a corundum stone mill, operated under the following conditions:

Gap=0.001 mm
Pressure/pump=18.7 kg/cm² (1 kg/cm²=0.98 bar)
Speed=19,300 R.P.M.
Discs grain coarses=180
Cooling mixture inlet temperature—5°C; outlet average temperature 20°C
Motor 380 V—3 phase current—50 Hz fire proof—80 HP (596.56×10² W).

The slurry was passed through the mill only once over a period of 36 minutes and at an inlet slurry temperature of 20°C and an outlet slurry temperature of 27°C.

The ground product obtained, after drying, had the following analysis:

Granulometry (by number):
100% less than 15 $\mu$m,
88% less than 10 $\mu$m,
48% less than 6 $\mu$m,
22% less than 3 $\mu$m;

Capacity:
Cl⁻=4.2 meq/g dry resin (USP=4 to 4.86 i.e. 14 to 17% chloride)
Glycocholate=2.2 g/g dry resin (USP=1.8 to 2.2).

Other characteristics:
Dialyzable quaternary ammonium salts=0.01% as benzyltrimethylammonium chloride
(USP spec 0.05% maximum)

Residue on ignition: 0.02% (USP 0.1%);
pH 5.3 (USP 4 to 6) in a slurry;
Heavy metals: 0.0015% (USP 0.0020%);
Loss on drying 8% (USP 5 to 12%);

Colour:     white;
Odour:      odourless;
Taste:      tasteless;
Solubility: insoluble in $H_2O$ alcohol, benzene, $CHCl_3$ and ether.

Comparative example

In this example another portion of the cholestyramine wet beads used in Example 1 was ground by conventional methods to pass through 200 US (0.074 mm) mesh. A comparison of the properties of this prior art resin with that of Example 1 is given in Table I.

TABLE I

|  | Prior art resin | Example 1 resin |
| --- | --- | --- |
| Colour | Buff-coloured | White |
| Odour | Slight amine like | Odourless |
| Taste | Gritty | Tasteless |
| Capacity Cl | 14.00% | 14.7% |
| Capacity glycocholate (g/g dry resin) | 1.83 | 2.20 |
| Dializable quat. salts | 0.048% | 0.010% |
| Residue on ignition | 0.02% | 0.02% |
| Heavy metals | 0.0015% | 0.0015% |
| pH | 5.5 | 5.3 |
| Loss on drying | 10.5% | 8% |

It will be apparent from this Table that the resin of Example 1 has not undergone the increase in dializable quaternary salts that would have been expected in reducing it to the smaller particle size, and furthermore that it has a higher glycocholate capacity than the standard resin.

Thus this invention makes it possible to produce cholestyramine resin of the particle size hereinbefore defined and having a content of dializable quaternary salts (as benzyltrimethylammonium chloride) less than 0.035%, preferably less than 0.025%. This is the result of the particle size reduction being carried out under conditions in which the resin does not become heated to the point of substantial degradation.

Another aspect of this invention therefore comprises cholestyramine resin, usually crosslinked styrene quaternary ammonium salt resin in the form of a non-toxic salt, such as chloride, sulphate, acetate or phosphate or in the free base form, having a particle size in the swollen state in which 90% by weight is below 30 $\mu$m, having a glycocholate capacity at least equal to the minimum specified in the U.S. Pharmacopeia and a maximum content of dializable quaternary ammonium salts, expressed as benzyltrimethylammonium chloride, of 0.035% by weight.

The maximum dializable quaternary ammonium salt content of the cholestyramine resins of this invention is below the maximum of 0.05% specified in the U.S. Pharmacopeia, and the other characteristics of the product at least conform to, and in most cases exceed, the U.S. Pharmacopeia minimum. This is evident from the Example above which also demonstrates the improved palatability of the cholestyramine resin of the invention.

The resins of the invention can be made into conventional pharmaceutical formulations such as tablets, ointments, creams, elixirs, syrups, emulsions, aqueous solutions or suspensions with added flavouring, or suspensions in organic diluents such as corn oil or mineral oil. The unit dosage is of convenient size such as tablets containing from 100 mg to 1 gram of the resin or suspensions containing from 100 mg to 1 g of resin per 5 cm³. Examples of suitable formulations are given in British Patent 929,391.

This invention also extends to such pharmaceutical formulations containing resin of the invention.

Example 2

40 kg of deionised sterile water were added to 200 kg of wet cholestyramine beads having an average particle size of 600 $\mu$m, a moisture content of 76%, an exchange capacity of 4.3 meq/g dry Cl⁻, and a specific gravity of 0.688 g/litre. The slurry was agitated for 5 minutes and left to stand for 20 minutes.

The slurry was kept under agitation and fed to a mill using a pressure slurry pump.

The mill used was a corundum stone mill operated under the following conditions:

| | |
|---|---|
| Gap: | 0.001 mm. |
| Pressure: | 4 kg/cm² (1 kg/cm²=0.98 bar) |
| Speed: | 3000 RPM |
| Grain of discs: (Rotor-stator) | 80—60 |
| Temperature: | about 20°C inlet |
| | about 70°C outlet |
| Motor: | As in Example 1. |

The slurry was passed through the mill once only over a period of 60 minutes. The ground product obtained had the following analysis (wet) using Coulter Counter equipment with:

| | |
|---|---|
| Aperture resistance: | 2—10 K$\Omega$ |
| Aperture diameter: | 280 mm Serial No. 25287 |
| Analysis (by number): | 90% less than 15 $\mu$m |
| | 65% less than 10 $\mu$m |
| | 20% less than 6 $\mu$m. |

**Claims**

1. A process for the comminution of particles of synthetic polymeric ion exchange or adsorbent resin wherein beads, or fragmented beads, thereof are swollen or shrunk by contact with water or an organic solvent in a slurry therewith to introduce strain within the resin particle and are subsequently ground, as a slurry, in a rotary attrition mill.

2. A process as claimed in Claim 1 wherein the rotary attrition mill shears and cuts the resin particles by passing them through a predetermined grinding gap between relatively rotating surfaces.

3. A process as claimed in any preceding claim wherein, after the swelling or shrinking step, the resin particles are comminuted in an other type of mill before being passed to the rotary attrition mill.

4. A process as claimed in any preceding claim wherein the swelling or shrinking is effected by contacting the resin particles for at least 0.5 minutes with the water or organic solvent.

5. A process as claimed in any preceding claim wherein the ratio of liquid to resin in the slurry is at least 0.3:1.

6. A process as claimed in Claims 4 or 5 wherein the contact time is at least sufficient to cause the beads to shrink to 95% or swell to 105% of their dry size.

7. A process as claimed in any preceding claim wherein the resin particles subjected to the swelling/shrinking step have a minimum particle size of 10 $\mu$m.

8. A process as claimed in any preceding claim wherein the grinding gap in the rotary attrition mill is 0.001 to 0.01 mm, the pressure is 2 to 40 kg/cm² (1 kg/cm²=0.98 bar) and the relative speed between the grinding surfaces is 2,000 to 25,000 R.P.M.

9. A process as claimed in any preceding claim wherein grinding is continued until the resin has a particle size such that at least 90% by weight and/or number is below 30 $\mu$m in average particle diameter in the wet swollen state.

10. A process as claimed in any preceding claim wherein the resin comprises cholestyramine.

11. A process as claimed in Claim 10 wherein the contact time in the swelling/shrinking step is at least 5 minutes.

12. Comminuted synthetic polymeric ion exchange or adsorbent resin obtainable by the process of claim 1, having a particle size such that at least 90% by weight and/or number is below 30 $\mu$m in average particle diameter in the wet swollen state.

13. Comminuted cholestyramine as claimed in Claim 12 having a glycocholate capacity of 1.8 to 2.2 g/g dry resin and a maximum content of dializable quaternary ammonium salts, expressed as benzyltrimethyl ammonium chloride, of 0.035% by weight.

**0 026 574**

14. Comminuted cholestyramine as claimed in Claim 13 having a particle size wherein at least 90% by weight is below 30 $\mu$m in average particle diameter in the wet swollen state.

15. Comminuted resin as claimed in any of Claims 12 to 14 in aqueous dispersion, or in pharmaceutically acceptable, orally injestible, unit dosage form, or in other pharmaceutical formulation.

**Revendications**

1. Procédé pour broyer des particules d'une résine polymère synthétique échangeuse d'ions ou adsorbante dans lequel on gonfle ou on rétracte des perles, ou des perles fragmentées, de cette résine par contact avec de l'eau ou un solvant organique dans laquelle ou lequel elles sont en dispersion, provoquant ainsi des tensions à l'intérieur des particules de résine qui sont ensuite broyées, à l'état de dispersion, dans un broyeur rotatif à frottements.

2. Procédé selon la revendication 1, dans lequel le broyeur rotatif à frottement cisaille et découpe les particules de résine au cours du passage de ces particules dans un intervalle de broyage déterminé entre des surfaces en rotation mutuelle.

3. Procédé selon l'une quelconque des revendications qui précèdent dans lequel, après l'opération de gonflement ou de rétractionou contraction, les particules de résine sont broyées dans un autre type de broyeur avant d'être envoyées au broyeur rotatif à frottement.

4. Procédé selon l'une quelconque des revendications qui précèdent dans lequel le gonflement ou la rétraction est provoqué par contact des particules de résine pendant au moins 0,5 minute avec l'eau ou le solvant organique.

5. Procédé selon l'une quelconque des revendication qui précèdent dans lequel le rapport du liquide à la résine dans la dispersion est d'au moins 0,3:1.

6. Procédé selon la revendication 4 ou 5, dans lequel la durée de contact est au moins suffisante pour que les perles se rétractent à 95% ou gonflent à 105% de leur dimension à l'état sec.

7. Procédé selon l'une quelconque des revendications qui précèdent dans lequel les particules de résine soumises à l'opération de gonflement/rétraction ont une dimension de particule minimum de 10 microns.

8. Procédé selon l'une quelconque des revendications qui précèdent dans lequel l'intervalle de broyage dans le broyeur rotatif à frottement est de 0,001 à 0,01 mm, la pression est de 2 à 40 kg/cm$^2$ et la vitesse relative entre les surfaces de broyage est de 2.000 à 25.00 tours/minute.

9. Procédé selon l'une quelconque des revendications qui précèdent dans lequel on poursuit le broyage jusqu'à ce que la résine ait une dimension de particule telle qu'au moins 90% en poids et/ou en nombre aient un diamètre de particule moyen inférieur à 30 microns à l'état mouillé et gonflé.

10. Procédé selon l'une quelconque des revendications qui précèdent dans lequel la résine comprend de la cholestyramine.

11. Procédé selon la revendication 10 dans lequel la durée de contact à l'opération de gonflement/rétraction est d'au moins 5 minutes.

12. Résine polymère synthétique échangeuse d'ions ou adsorbante broyée susceptible d'être obtenue par le procédé selon la revendication 1, ayant une dimension de particule telle qu'moins 90% en poids et/ou en nombre aient un diamètre de particule moyen inférieur à 30 microns à l'état mouillé gonflé.

13. Cholestyramine broyée selon la revendication 12 ayant une capacité de glycocholate de 1,8 à 2,2 g par gramme de résine sèche et une teneur maximum en sels d'ammonium quaternaire dialysables, exprimée en chlorure de benzyltriméthyl-ammonium, de 0,035% en poids.

14. Cholestyramine broyée selon la revendication 13 ayant une dimension de particule pour laquelle au moins 90% en poids ont un diamètre de particule moyen inférieure à 30 microns à l'état mouillé gonflé.

15. Résine broyée selon l'une quelconque des revendications 12 à 14 en dispersion aqueuse ou sous forme d'unités de dosage acceptables pour l'usage pharmaceutique, ingérables par voie orale, ou sous une autre forme pharmaceutique.

**Patentansprüche**

1. Verfahren zur Zerkleinerung von Teilchen aus einem synthetischen polymeren Ionenaustauscher- oder Adsorbensharz, dadurch gekennzeichnet, daß Kügelchen oder fragmentierte Kügelchen des Harzes durch Kontakt mit Wasser oder einem organischen Lösungsmittel in einer Aufschlämmung zur Einführung einer Spannung in das Harzteilchen gequollen oder geschrumpft und anschließend als Aufschlämmung in einer Rotationszerkleinerungsmühle vermahlen werden.

2. Verfahren nach Anspruch 1, dadurch gekennzeichnet, daß die Rotationsmühle die Harzteilchen in der Weise einer Scherwirkung unterzieht und schneidet, daß sie durch einen vorbestimmten Mahlspalt zwischen sich relativ zueinander drehenden Oberflächen geführt werden.

3. Verfahren nach einem der vorhergehenden Ansprüche, dadurch gekennzeichnet, daß nach der Quell- oder Schrumpfstufe die Harzteilchen in einem anderen Mühlentyp zerkleiner werden, bevor sie der Rotationszerkleinerungsmühle zugeführt werden.

6

4. Verfahren nach einem der vorhergehenden Ansprüche, dadurch gekennzeichnet, daß die Quellung oder Schrumpfung durch Kontaktieren der Harzteilchen während wenigstens 0,5 min mit Wasser oder einem organischen Lösungsmittel durchgeführt wird.

5. Verfahren nach einem der vorhergehenden Ansprüche, dadurch gekennzeichnet, daß das Verhältnis von Flüssigkeit zu dem Harz in der Aufschlämmung wenigstens 0,3:1 beträgt.

6. Verfahren nach Anspruch 4 oder 5, dadurch gekennzeichnet, daß die Kontaktzeit wenigstens dazu ausreicht, daß die Kügelchen auf 95% ihrer Trockengröße schrumpfen oder auf 105% ihrer Trockengröße quellen.

7. Verfahren nach einem der vorhergehenden Ansprüche, dadurch gekennzeichnet, daß die Harzteilchen, die der Quell-/Schrumpf-Stufe unterzogen werden, eine minimale Teilchengröße von 10 $\mu$m besitzen.

8. Verfahren nach einem der vorhergehenden Ansprüche, dadurch gekennzeichnet, daß der Mahlspalt in der Rotationszerkleinerungsmühle 0,001 bis 0,01 mm, der Druck 2 bis 40 kg/cm$^2$ (1 kg/cm$^2$=0,98 bar) und die relative Geschwindigkeit zwischen den Mahloberflächen 2.000 bis 25.000 Upm (R.P.M.) betragen.

9. Verfahren nach einem der vorhergehenden Ansprüche, dadurch gekennzeichnet, daß das Mahlen fortgesetzt wird, bis das Harz eine solche Teilchengröße besitzt, daß wenigstens 90% (bezogen auf das Gewicht und/oder die Anzahl) unterhalb 30 $\mu$m bezüglich des durchschnittlichen Teilchendurchmessers in feuchtem gequollenen Zustand vorliegen.

10. Verfahren nach einem der vorhergehenden Ansprüche, dadurch gekennzeichnet, daß das Harz aus cholestyramin besteht.

11. Verfahren nach Anspruch 10, dadurch gekennzeichnet, daß die Kontaktzeit während der Quell-/Schrumpf-Stufe wenigstens 5 min beträgt.

12. Zerkleinertes synthetisches polymeres Ionenaustauscheroder Adsorbensharz, dadurch gekennzeichnet, daß es nach dem Verfahren von Beispiel 1 erhältlich ist und eine derartige Teilchengröße besitzt, daß wenigstens 90%, bezogen auf das Gewicht und/oder die Anzahl, unterhalb 30 $\mu$m bezüglich des durchschnittlichen Teilchendurchmessers in feuchtem gequollenen Zustand vorliegen.

13. Zerkleinertes Cholestyramin gemäß Anspruch 12 mit einer Glycocholatkapazität von 1 g bis 22 g/g Trockenharz und einem maximalen Gehalt an dialysierbaren quaternären Ammoniumsalzen, ausgedrückt als Benzyltrimethylammoniumchlorid, von 0,035 Gew.-%.

14. Zerkleinertes Cholestyramin gemäß Anspruch 13 mit einer solchen Teilchengröße, daß wenigstens 90%, bezogen auf das Gewicht, unterhalb 30 $\mu$m bezüglich des durchschnittlichen Teilchendurchmessers in feuchtem gequollenen Zustand vorliegen.

15. Zerkleinertes Harz nach einem der Ansprüche 12 bis 14 in wäßriger Dispersion oder in pharmazeutisch verträglicher oral verabreichbarer Einheitsdosierungsform oder in Form einer anderen pharmazeutischen Formulierung.